Europäisches Patentamt

(19) European Patent Office

Office européen des brevets

(11) EP 0 450 922 B1

## (12) EUROPEAN PATENT SPECIFICATION

(45) Date of publication and mention
of the grant of the patent:
**07.07.1999 Bulletin 1999/27**

(51) Int. Cl.[6]: **A61L 15/00**, C08F 8/14,
C08F 8/30, C08F 8/32,
C08J 3/00, C08J 3/12

(21) Application number: **91302894.0**

(22) Date of filing: **02.04.1991**

(54) **Method for production of fluid stable aggregate**

Verfahren zur Herstellung von flüssigkeitsstabilem Aggregat

Procédé de préparation d'un agrégat stable à la fluidité

(84) Designated Contracting States:
**AT BE CH DE DK ES FR GB GR IT LI LU NL SE**

(30) Priority: **02.04.1990 US 502737**

(43) Date of publication of application:
**09.10.1991 Bulletin 1991/41**

(73) Proprietor:
**NIPPON SHOKUBAI KAGAKU KOGYO CO. LTD.**
**Osaka-shi, Osaka-fu 541 (JP)**

(72) Inventors:
• **Hatsuda, Takumi**
**Himeji-shi, Hyogo-ken (JP)**
• **Kimura, Kazumasa**
**Ikoma-gun, Nara-ken (JP)**
• **Nagauna, Kinya**
**Himeji-shi, Hyogo-ken (JP)**
• **Yano, Akito**
**Himeji-shi, Hyogo-ken (JP)**

(74) Representative:
**Rees, David Christopher et al**
**Kilburn & Strode**
**20 Red Lion Street**
**London WC1R 4PJ (GB)**

(56) References cited:
**EP-A- 0 238 050**          **EP-A- 0 317 106**
**EP-A- 0 318 989**          **GB-A- 2 162 525**
**US-B- 494 440**

## Description

Field of the Invention:

This invention relates to a fluid stable aggregates having no collapse of aggregate structure, wherein a plurality of absorbent resin powder particles are interparticle cross-linked to each other by covalent bonds, and a method for the production thereof.

Description of the Prior Art:

Attempts have been made heretofore to use an absorbent resin as one of the component materials for such sanitary articles as sanitary napkins and disposable diapers which function to absorb body fluids. Absorbent resins of this nature heretofore known to the art include, a hydrolyzed starch-acrylonitrile graft polymer (U.S. Patent No. 3,661,815), a neutralized starch-acrylic acid graft polymer (U.S. Patent No. 4,076,663), a saponified vinyl acetate-acrylic ester copolymer (Japanese Patent Laid-Open SHO 52(1977)-14,689), a hydrolyzed acrylonitrile copolymer or acrylamide copolymer (Japanese Patent Publication SHO 53(1978)-15,959), cross-linked products thereof, a partially neutralized polyacrylic acid, and a partially neutralized cross-linked polyacrylic arid (Japanese Patent Laid-Open SHO 57(1982)-34,101).

Characteristic properties of such absorbent resins include high absorption capacity, high absorption rate, liquid permeability, and large gel strength. However, it is difficult to improve such properties at the same time.

Attempts have been made to increase the absorption rate by decreasing the particle size of the absorbent resin, granulating the absorbent resin, or forming the absorbent resin in flakes. Generally, when the absorbent resin is formed in a small particle size, the resin particles on contact with urine convert themselves into what resembles wetted clusters of flour to an extent of lowering the absorption rate. When the absorbent resin is formed in the form of granules, there ensues a phenomenon that the granules themselves are independently converted into wetted clusters on contact with urine and the absorption rate is rather lowered. When the absorbent resin is formed in flakes the absorption rate is fairly improved; however, because of the occurrence of gel blocking, the absorption rate is not sufficient and the production of the absorbent has a restriction from the standpoint of process because the formation of the absorbent resin in flakes is not economical because the produced absorbent resin inevitably becomes bulky and necessitates larger facilities for transportation and storage.

On the other hand, a technique for enhancing the absorption rate and gel strength after absorption without decreasing absorption capacity is by cross-linking molecular chains near the surface of the absorbent resin surface crosslinking.

These techniques have been disclosed in Japanese Patent Laid-Open SHO 57(1982)-44,627, Japanese Patent Laid-Open SHO 58(1983)-42,602, Japanese Patent Publication SHO 60(1985)-18,609, U.S. Patent No. 4,666,983, U.S. Patent No. 4,497,930 and U.S. Patent No. 4,734,478, for example.

Most of the absorbent resins produced by these techniques, however, highly contain fine powder which passes the standard sieve of 100 mesh. In their actual use, they have encountered the following problems.

(1) They are liable to induce drift of dust and consequently entail impairment of the working environment and loss of weight.
(2) They exhibit poor mixability and dispersibility when they are mixed with other substances.
(3) Especially when the amount of fines is large, they are liable, on contact with a liquid, to gel block and suffer from residual liquid-permeability.
(4) Since the absorbent resins containing fine powder are deficient in flowability, they are liable to cause the phenomena of bridge formation and flushing in the hopper.

The methods proposed for the solution of this problem are the method which resorts to removal of the fine powder portion of the absorbent resin and the water agglomeration of the absorbent resin. The former method is not desirable because of its economic disadvantage.

Concerning the agglomeration by the use of water, the method is disclosed in U.S. Patent NO. 4,734,478, and comprises uniformly mixing an absorbent resin powder and water by the use of a specific mixing device such as a high-speed rotary paddle type mixer or an air current type mixer and then pulverizing and granulating the resultant mixture.

The method of water agglomeration (size enlargement) of the absorbent resin powder allows the absorbent resin powder to acquire improved handling as a particulate substance. However, the agglomerates thus obtained merely bind the powder to each other physically, so that the strength of agglomerates is very low. The agglomerates of absorbent resin obtained thereby are disintegrated into minute particles during their handling at a plant or in transit. If such agglomerates can maintain the agglomerate structure until it is subjected to use as the final product, it cannot be expected to maintain the agglomerate structure when the final product is used. For example, when the agglomerates of the absorbent resin are used in a disposable diaper, the agglomerates on contact with an aqueous liquid are disintegrated into their former basic particles of a small size and these particles are liable to form wetted clusters because of their small size. Further, the basic particles of the gel to be formed in consequence of absorption of aqueous liquid have a small size and, therefore, clog the capillaries in the pulp fibers and tend to induce the phenomenon of gel blocking. That is, the liquid permeability is reduced.

It has been discovered that the use of fluid stable aggregates in an absorbent, hydrogel-forming, polymer composition dramatically improves the performance of such polymer compositions. The fluid stable aggregates are capable of absorbing large quantities of liquids, have a rapid swelling rate, and retain their shape upon absorption of liquids so as to reduce the gel blocking effect of the overall polymer composition while increasing the absorptive rate.

The swelling rate of the polymer material refers to the average rate of fluid uptake to a given load by a sample of hydrogel-forming polymer material. Swelling rate is a measure of the permeability of the overall general mass as modified by the diffusion rate (gel blocking). Thus, the permeability of the gel becomes the limiting factor by limiting how fast free fluid can get to other particles in the mixture. Swelling rate is measured and defined in terms of grams of synthetic urine per gram of hydrogel-forming polymer per second. The swelling rate may be determined by using a test method as hereinafter described.

Because of the desirability of utilizing fluid stable aggregates in polymer compositions, it is desirable to find a method for producing such fluid stable aggregates. In addition, it is desirable to find a method to make fluid stable aggregates in an economic and efficient manner.

Thus, it is an object of the present invention to provide a method for producing fluid stable aggregates.

It is a further object of the present invention to provide a method for economically and efficiently producing fluid stable aggregates.

It is an even further object of the present invention to provide a method for producing polymer compositions containing relatively large quantities of fluid stable aggregates and having high swelling rate.

It is a still further object of the present invention to provide a method for producing polymer compositions having low fine powder content.

SUMMARY OF THE INVENTION

The objects described above are accomplished by a method for the production of a fluid stable aggregate, which comprises mixing (A) 100 parts by weight of an absorbent resin powder possessing a carboxyl group, (B) 0.01 to 30 parts by weight of a cross-linking agent having at least two functional groups capable of reacting with the carboxyl group of the absorbent resin powder, (C) 0 to 50 parts by weight of water, and (D) 0 to 60 parts by weight of a hydrophilic organic solvent in a high-speed stirring type mixer; the mixer having an inner surface formed substantially of a substrate (I) possessing a contact angle of not less than about 60° with respect to water and a heat distortion point of not lower than about 70°C under stirring conditions in which the leading-end of a stirring blade has a peripheral speeding not less than 600 m/min, and thereafter completing reaction of the absorbent resin powder (A) with the cross-linking agent (B) at a temperature in the range of 90° to 250°C under the condition that total kinetic energy F added to the mixture during the reaction is satisfied by the following equation:

$$0 \leqq F \leqq 36,000 \text{ joule/kg}$$

and in which the kinetic energy Fa per minute added during the reaction is not more than 600 joule/kg.

According to the present invention, mixing of the absorbent resin powder (A) with the cross-linking agent (B) is carried out by the above-mentioned high-speed stirring type mixer, and the kinetic energy added during the reaction is in the range of the above mentioned range, so the polymer compositions thus obtained contains very little fine powder and has high swelling rate, and relatively large quantities of fluid stable aggregates. Such polymer compositions are not only enhanced in crosslinking degree on the surface region of the absorbent resin powder (A), but also contain a large amount of strong aggregates having a plurality of the absorbent resin powders (A) inter-particle crosslinked by covalent bonds with the crosslinking agent (B), so the polymer composition having less occurrence of the fine powder can be obtained and when the polymer composition contacts with water, there is no collapse of the aggregate and gel blocking does not occur. When these polymer compositions are dispersed among pulp fibers and, in this state, exposed to an aqueous liquid and consequently swelled and gelled, it manifests an effect of expanding the capillaries among the pulp fibers instead of clogging them. When these polymer compositions are used in a diaper, for example, the diaper enjoys satisfactory liquid permeability and has reduced leakage. The fluid stable aggregate thus obtained may be used for sanitary field such as disposable diaper, sanitary napkins and disposable towels, for civil engineering field such as water sealing agents, dewproofing agents and sludge coagulating agents, for architectural field such as humidity controlling agents, for agricultural and horcultural field such as seed and seedling preserving sheets, for foodstuff packaging field such as freshness preserving materials, dehydrating agents and desiccants, for medical field such a s blood absorbents and surgical sponges, for electrical field such as water sealant for cables and humidity sensors, and other oil-water separating agents, sweat absorbents, water swellable toys, ion exchenging resins, and they can absorb aqueous liquids such as water, urine, blood, steam, meat juices, ion-containing water including sea water, aqueous solutions dispersing organics, etc.

BRIEF DESCRIPTION OF THE DRAWING

Fig. 1 is a sectional view of an embodiment of a mixer used in the present invention, and
Fig. 2 is a sectional view of an another mixer used in the present invention.

EXPLANATION OF THE PREFERRED EMBODIMENT

The absorbent resin powder (A) to be used in the present invention is required to possess a carboxyl group. The absorbent resin powders which answer this description include hydrolyzed starch-acrylonitrile graft copolymer, partially neutralized starch-acrylonitrile graft copolymer, saponified vinyl acetate-acrylic ester copolymers, hydrolyzed acrylonitrile copolymers or acrylamide copolymers, cross-linked products of the copolymers, partially neutralized polyacrylic acid, and cross-linked product of partially neutralized polyacrylic acid which are invariably in a powdered form, for example. These absorbent resin powders may be used either independently or in the form of a mixture of two or more members. Though the absorbent resin powder (A) is preferable to possess a crosslinked structure, it may be used effectively in a form destitute of such a cross-linked structure.
In the various absorbent resin powders (A) mentioned above, those which prove to be particularly desirable are the absorbent resins to be shown below in (1) to (5).

(1) The powdery alkali metal acrylate polymer obtained by thermally drying a gel-like water-containing polymer formed by copolymerizing 100 parts by weight of an acrylic acid salt monomer comprising 1 to 50 mol% of acrylic acid and 99 to 50 mol% of an alkali metal acrylate and 0 to 5 parts by weight of a cross-linking monomer in an aqueous solution having a monomer concentration of not less than 20% by weight.
(2) The powdery resin obtained by dispersing the aqueous solution of acrylic acid and/or an alkali metal acrylate containing a water-soluble radical polymerization initiator and optionally a cross-linking monomer in an alicyclic and/or aliphatic hydrocarbon solvent in the presence of a surfactant possessing HLB in the range of 8 to 12 and suspension polymerizing the resultant dispersion.
(3) The powdery saponified copolymer of a vinyl ester with an ethylenically unsaturated carboxylic acid or a derivative thereof.
(4) The powdery absorbent resin obtained by polymerizing starch and/or cellulose, a monomer possessing a carboxyl group, or capable of forming a carboxylic group in consequence of hydrolysis and optionally a cross-linking monomer in an aqueous medium and optionally further hydrolyzing the resultant polymer.
(5) The powdery absorbent resin obtained by causing an alkali substance to react upon maleic anhydride copolymer comprising maleic anhydride and at least one monomer selected from the group consisting of $\alpha$-olefins and vinyl compounds and optionally causing a polyepoxy compound to react with the resultant reaction product.

Though the amount of the carboxyl group possessed by the absorbent resin powder (A) is not specifically limited, the carboxyl group is preferable to be present in an amount of not less than 0.01 equivalent, based on 100 g of the absorbent resin powder (A). In the case of a partially neutralized polyacrylic acid, for example, the proportion of the unneutralized polyacrylic acid is preferable to be in the range of 1 to 50 mol%, preferably 5 to 40 mol%.
The shape of particles of the absorbent resin powder (A) to be used in the present invention is not specifically limited. It may be the sphere shape obtained by the reverse-phase suspension polymerization, the flake shape obtained by drum drying, or the irregular shaped particles obtained by pulverizing resin clusters.
The cross-linking agents (B) which are usable in the present invention include compounds possessing in the molecular unit thereof at least two functional groups capable of reacting with the carboxyl group. The compounds which are usable as the cross-linking agent (B) in the present invention include polyhydric alcohols such as ethylene glycol, diethylene glycol, triethylene glycol, tetraethylene glycol, polyethylene glycol, glycerol, propylene glycol, diethanol amine, triethanol amine, polyoxy propylene, oxyethylene-oxypropylene block copolymer, pentaerythritol, and sorbitol, polyglycidyl ether compounds such as ethylene glycol diglycidyl ether, polyethylene glycol diglycidyl ether, glycerol polyglycidyl ether, diglycerol polyglycidyl ether, polyglycerol polyglycidyl ether, sorbitol polyglycidyl ether, pentaerythritol polyglycidyl ether, propylene glycol diglycidyl ether, and polypropylene glycol diglycidyl ether, haloepoxy compounds

4

such as epichlorohydrin and α-methylfluorohydrin, polyamine compounds such as ethylene diamine, diethylene triamine, triethylene tetramine, tetraethylene pentamine, pentaethylene hexamine, and polyethylene imine, for example. One cross-linking agent or two or more mutually unreactive cross-linking agents selected from the group mentioned above may be used.

Among other compounds mentioned above, it is particularly desirable to use at least one compound selected from the group consisting of diethylene glycol, triethylene glycol, polyethylene glycol, glycerol, polyglycerol, propylene glycol, diethanol amine, triethanol amine, polyoxy propylene, oxyethylene-oxypropylene block copolymer, sorbitan fatty acid esters, polyoxyethylene sorbitan fatty acid esters, trimethylol propane, pentaerythritol, and sorbitol.

The proportion of the cross-linking agent (B) to be used in this invention is in the range of 0.01 to 30 parts by weight, preferably 0.1 to 10 weight. So long as this proportion is in this range, the produced polymer composition has a high content of fluid stable aggregates and a high swelling rate. If the proportion exceeds 30 parts by weight, the excess is wasted without producing any economic effect and suffered to overburden the accomplishment of a proper cross-linking effect and decrease the absorption capacity of the produced absorbent. Conversely, if this proportion is less than 0.01 part by weight, the effect of this invention is attained only with difficulty.

In the present invention, water (C) may be used during the mixing of the absorbent resin powder (A) with the cross-linking agent (B). The water (C) functions not only to promote uniform dispersion of the cross-linking agent (B) on the surface of the absorbent resin powder (A) and permeation of the cross-linking agent (B) in the surface region of the particles of the absorbent resin powder (A) but also to promote the cross-linking reaction between the particles of the absorbent resin powder (A).

The fluid stable aggregate in accordance with the present invention is preferably produced by adding water (C) during mixing the absorbent resin powder (A) with the crosslinking agent (B) compared with the case without addition of the water (C). That is, in the present invention, the water (C) is used in the range of 0 to 50 parts by weight, preferably in the range of 0.5 to 40 parts by weight, more preferably 2 to 40 parts by weight, based on 100 parts by weight of the absorbent resin powder (A), depending on the kind and particle size of the absorbent resin powder (A). If the amount of water (C) exceeds 50 parts by weight, the heat treatment consumes an unduly long time and the crosslinking agent (B) is caused to permeate to the cores of the particles of the absorbent resin powder (A) and the absorption capacity of the produced fluid stable aggregate will decrease too much. Further, the absorbent resin powder (A) tends to form wetted clusters and the mixing will not be uniform.

The hydrophilic organic solvent (D) which is optionally used in the present invention is only required to be such that it will uniformly mix with the cross-linking agent (B) and refrain from producing any adverse effect upon the quality of the absorbent resin powder (A). The hydrophilic organic solvents which answer this description include lower alcohols such as methanol, ethanol, n-propanol, isopropanol, n-butanol, isobutanol, sec-butanol, and t-butanol, ketones such as acetone, methylethyl ketone, and methylisobutyl ketone, ethers such as dioxane, tetrahydrofuran, and diethyl ether, amides such as N,N-dimethyl formamide and N,N-diethyl formamide, and sulfoxides such as dimethyl sulfoxide, for example. The hydrophilic organic solvent (D) functions to effect uniform dispersion of the cross-linking agent (B) and the optionally used water (C) on the surface of the absorbent resin powder (A).

The amount of the hydrophilic organic solvent (D) to be used in the present invention is in the range of 0 to 60 parts by weight, preferably 0.1 to 10 parts by weight, based on 100 parts by weight of the absorbent resin powder (A), though variable with the kind and particle size of the absorbent resin powder (A) to be used. If the amount of the hydrophilic organic solvent (D) exceeds 60 parts by weight, the excess is not observed to give a proportionate addition to the effect aimed at but is suffered to impair the economy by increasing the amount of energy to be spent for the purpose of drying. For this invention, the use of the hydrophilic organic solvent (D) is not always necessary because the mixing of the absorbent resin powder (A) with the cross-linking agent (B) is carried out by the use of a specific high-speed stirring type mixer which will be described more fully hereinafter. There are times when the use of the hydrophilic organic solvent (D) will result in an enhanced effect of this invention, depending on the kind and amount of the cross-linking agent (B) or on the amount of water (C) or the kind and particle size of the absorbent resin powder (A) to be used. When mixing of the absorbent resin powder (A) with the crosslinking agent (B) and, if necessary water (C) is insufficient, e.g., when particle size of the absorbent resin powder (A) is too small or when an amount of water to be used is too large compared to the amount of the crosslinking agent (B), additional use of a comparatively small amount of an organic solvent makes it easy to obtain the effect of the present invention.

In this invention, the mixing of the absorbent resin powder (A) with the cross-linking agent (B) is carried out by the use of a high-speed stirring type mixer.

The high-speed stirring type mixer to be used in this invention is capable of rotating the stirring blades thereof at a leading-end peripheral speed of not less than 600 m/minute, preferably in the range of 1,000 to 3,000 m/minute. If the leading-end peripheral speed of the stirring blades is less than 600 m/min, the absorbent resin powder (A) cannot be thoroughly mixed with such amounts of the cross-linking agent (B), water (C), and the hydrophilic organic solvent (D), and the inter-particle crosslinking of the absorbent resin powder (A) to each other by the crosslinking agent (B) becomes inefficient in a commercial process, as required to achieve the effect of this invention. If the leading-end peripheral speed of the stirring blades exceeds 3,000 m/minute, the impact of the stirring induces fracture of the absorbent resin powder (A).

The high-speed stirring type mixer to be used in this invention has at least one stirring shaft having at least one stirring blade, and the stirring shaft can be rotated at not less than about 600 m/minute, preferably 1,000 to 3,000 m/minute of peripheral speed of the leading-end of the stirring blade.

High-speed stirring type mixers include mixers of the type provided on the bottom inside a stirring tank thereof with rotary blades such as, for example, Henschel Mixer [produced by Mitsui Miike Machinery Co., Ltd.], New Speed Mixer [produced by Okada Seiko K.K.] and Heavy-Duty Matrix [produced by Nara Kikai Seisakusho K.K.] and mixers of the type capable of (continuously) mixing two or more kinds of powder or a powder with a liquid by the high-speed rotation of a rotar provided with a multiplicity of paddles and disposed inside a cylindrical container such as, for example, Turbulizer and Sand Turbo (both produced by Hosokawa Micron K.K.). Among these high-speed stirring type mixers, a continuous type mixer has high productivity and is preferable.

The high-speed stirring type mixer to be used in this invention is provided with an inner surface formed substantially of a substrate (I) possessing a contact angle of not less than about 60° with respect to water and a heat distortion point of not lower than about 70°C in order to obtain sufficient mixing effect of the absorbent resin powder (A) with the crosslinking agent (B).

Especially in the present invention when water (C) is used in an amount of higher region within the range, the mixing of the absorbent resin powder (A) with the crosslinking agent (B) and water (C) becomes insufficient, unless the method is carried out under the above-mentioned conditions. In such a case, the mixing property is sometimes improved by using a mixer having an inner wall of a substrate (I) possessing a contact angle of not less than 60°. If the heat distortion point is lower than about 70°C, the substrate (I) is incapable of withstanding the heat to be generated during the course of mixing, with the result that no stable mixing can be continued.

The substances which are usable as the substrate (I) for the formation of the inner surface of the mixer include synthetic resins such as polyethylene, polypropylene, polyester, polyamide, fluorine resin, polyvinyl chloride, epoxy resin, and silicone resin and the synthetic resins mentioned above which are complexed and reinforced with inorganic fillers such as glass, graphite, bronze, and molyodenum disulfide and organic fillers such as polyimide, for example. Among other substances mentioned above, fluorine resins such as polyethylene tetrafluoride, polyethylene trifluoride, polyethylene trifluorochloride, ethylene tetrafluoride-ethylene copolymer, ethylene trifluorochloride-ethylene copolymer, propylene pentafluoride-ethylene tetrafluoride copolymer, perfluoroalkylvinyl ether-ethylene tetrafluoride copolymer, polyvinylidene fluoride, and polyvinyl fluoride, for example, are particularly preferable.

The high-speed stirring type mixer to be used in this invention may have the mixer itself formed of the substrate (I) mentioned above. Generally, the mixer is formed of a metal material and has the inner wall thereof lined with a coating of the substrate (I) or covered with a sleeve of the substrate (I).

Preferably, a shaped material, more preferably a shaped cylindrical material comprising the substrate (I) is inserted into the high-speed stirring type mixture.

Further, the shaped material of the substrate (I) has preferably not less than 5 mm, more preferably not less than 10 mm of thickness. When the absorbent resin powder (A) is mixed with the cross-linking agent (B) for a long time, if the mixer wherein the inner surface of the mixer is coated with the substrate (I) is used, the layer of the substrate (I) is defaced because of insufficient thickness within a comparatively short time and the foundation appears, so the mixing becomes unstable. In addition, the coating layer requires more time and cost when it is repaired. On the contrary, when the shaped material of the substrate (I) having not less than 5 mm of the thickness is detachably inserted into the mixer, the mixture can be stably obtained even for a long time, and the repairing can be easily carried out.

When the absorbent resin powder (A) with the crosslinking agent (B), and if necessary, water (C) and the hydrophilic organic solvent (D) is mixed in this invention, it is preferred to be carried out using the high-speed stirring type mixer under the above mentioned condition, and if it is not carried out under this condition, the object of this invention is not be attained. The reason is not clear, but it is thought that a specific bond occur between the absorbent resin powders (A) and reaction efficiency between the particles becomes high by the fact that mixing is carried out by strong shearing force in addition to the fact mixing of the absorbent resin powder (A) with the crosslinking agent (B) is carried out homogeneously.

In the present invention, although the reaction between the absorbent resin powder (A) and the crosslinking agent (B) can be carried out under stirring or non-stirring condition, it is necessary that total kinetic energy F loaded to a mixture of the absorbent resin powder (A), the crosslinking agent (B), and if necessary water (C), the hydrophilic organic solvent (D), and a water-insoluble fine powder (E) to be explained below until the reaction of the absorbent resin powder (A) with the crosslinking agent (B) is completed satisfies the following equation:

$$0 \leqq F \leqq 36,000 \text{ joule/kg}$$

wherein the kinetic energy Fa per minute added during the reaction is not more than 600 joule/kg.

That is, the fluid stable aggregate obtained by the method of the present invention is formed by interparticle crosslinking of a plurality of the absorbent resin powder (A) through the reaction of the absorbent resin powder (A) with the crosslinking agent (B). Therefore, if excess kinetic energy is added before the completion of the reaction of the absorbent resin powder (A) with the crosslinking agent (B), the weak agglomeration structure based on physical bond is broken, so the fluid stable aggregate to be aimed cannot be obtained. The most ideal state resides in that the plurality of the absorbent resin powder (A) is maintained at a physical contact state at the same contact point during the reaction of the absorbent resin powder (A) with the crosslinking agent (B).

If too much kinetic energy is added to the mixture of the absorbent resin powder (A) and the crosslinking agent (B) prior to the completion of the crosslinking reaction of the absorbent resin powder (A) with the crosslinking agent (B), the polymer composition having high fluid stable aggregate content, very little fine powder content and an excellent swelling rate will not be obtained.

The term of the "total kinetic energy" in this specification is a kinetic energy loaded to a mixture of the absorbent resin powder (A), the crosslinking agent (B) and, if necessary, water (C), the hydrophilic organic solvent (D), and water-insoluble fine powder (E) during the term from charging the mixture into a reactor to completion of the reaction. Further the "total kinetic energy" is mainly the energy to be loaded for stirring the mixture and for example, the energy used for varying the position of a bucket containing the mixture is not included.

If the above mentioned conditions are satisfied, an ordinary drier or heating furnace can be used as a heat treatment device that is reactor to be used for obtaining the fluid stable aggregate of the present invention.

The heat treatment can be carried out by the use of an ordinary drier or heating furnace. The driers which are usable for the heat treatment include a horizontal stirring drier, rotary drier, disc drier, a fluidized-bed drier, an air-current drier, and an infrared drier, for example. The heat treatment may be started immediately after the completion of the mixing or after the product of the mixture has been left standing for a prescribed time.

In the present invention, when the absorbent resin powder (A) and the cross-linking agent (B) are mixed by the use of the high-speed stirring type mixer, they may be mixed in conjunction with a water-insoluble fine powder (hereinafter referred to as "powder (E)"). The additional use of the powder (E) serves the purpose of amply heightening the effect of mixing and improving the effect of agglomeration. Therefore, the reaction product, polymer composition containing a large amount of the fluid stable aggregate, is superior in swelling rate.

The water-insoluble fine powders (E) which are usable herein include organic powders such as carbon black and activated carbon which are effective in improving the lightfastness of the absorbent resin and also capable of producing an odorizing effect, and pulp powder, and inorganic powders ,such as talc, pyrophylite, kaolinite, hulsite, and other similar clay minerals, and fine silica such as Aerosil 200 (produced by Nippon Aerosil K.K.) comprised mainly of silicon dioxide particles having an average particle size of not more than 50 $\mu$m, and carplex #80 (produced by Shionogi & Co., Ltd.), for example.

Particle diameter of these water-insoluble fine powders (E) is not more than 1,000 $\mu$m, preferably not more than 100 $\mu$m, more preferably not more than 50 $\mu$m.

The amount of the water-insoluble fine powder (E) to be used is in the range of 0.01 to 10 parts by weight, preferably 0.01 to 5 parts by weight, based on 100 parts by weight of the absorbent resin powder (A). So long as the amount is in the range mentioned above, the polymer composition which has a high swelling rate, a high fluid stable aggregate content, and has a sufficiently low fine powder content can be obtained efficiently. If this amount exceeds 10 parts by weight, the excess does not produce any proportionate addition to the effect but rather impairs the absorption capacity and at times renders the agglomeration difficult.

When the absorbent resin powder (A) and the cross-linking agent (B) are mixed in conjunction with the water-insoluble fine powder (E), this powder (E) may be directly fed to the high-speed stirring type mixer similarly to the absorbent resin powder (A) and the cross-linking agent (B) so as to participate in the mixing from the beginning. Optionally, the powder (E) may be premixed with the abosrbent resin powder (A) in a varying mixer and then the resultant premix and the cross-linking agent (B) may be fed to the high-speed stirring type mixer to be mixed therein. Alternatively, the powder (E) may be mixed with the crosslinking agent (B) to obtain a mixture, and then the mixture may be mixed with the absorbent resin powder (A). The mixing may be carried out in the presence of water (C) and/or the hydrophilic organic solvent (D). At times the strength of bonds between particles of the absorbent resin powder (A) increases by using the water-insoluble fine powder (E).

Further, when the absorbent resin powder (A) is mixed with the crosslinking agent (B), the effect of this invention is enhanced by reacting the absorbent resin powder (A) with the cross-linking agent (B) until the reaction is completed. The reaction may be considered completed when Q reaches a value so that one of the following equation (a-1), (a-2), (b-1) and (b-2) is satisfied :

(wherein the water-insoluble fine powder (E) is not used during the course of mixing)

$$30 \leqq \frac{(100 + R)}{100} \times \frac{Q}{P} \times 100 \leqq 95 \qquad \text{(a-1)}$$

preferably

$$40 \leq \frac{(100 + R)}{100} \times \frac{Q}{P} \times 100 \leq 85 \qquad \text{(a-2)}$$

wherein P is absorption capacity of absorbent resin powder (A) using physiological saline solution, Q is absorption capacity of the reaction product using physiological saline solution, and R is the amount, in parts by weight, of crosslinking agent (B) to be used based on 100 parts by weight of absorbent resin powder (A), or

(wherein the water-insoluble fine powder (E) is used during the course of mixing)

$$30 \leq \frac{(100 + R + S)}{100} \times \frac{Q}{P} \times 100 \leq 95 \qquad \text{(b-1)}$$

preferably

$$40 \leq \frac{(100 + R + S)}{100} \times \frac{Q}{P} \times 100 \leq 85 \qquad \text{(b-2)}$$

wherein P is absorption capacity of abosrbent resin powder (A) using physiological saline solution, Q is absorption capacity of the reaction product using physiological saline solution, R is the amount, in parts by weight, of cross-linking agent (B) to be used based on 100 parts by weight of absorbent resin powder (A), and S is the amount, in parts by weight, of water-insoluble fine powder (E) to be used based on 100 parts by weight of absorbent resin powder (A).

If the calculation value of the above equation (a-1) or (b-1) is not less than 95, only cross-link density of the surface region of the abosrbent powder (A) is increased, so enhancement of the gel strength based on increase of the cross-link density of the surface region is recognized, but the polymer composition having a certain level or more of swelling rate and fluid stable aggregate content is difficult to be obtained. On the other hand, if the calculation value of the above equation (a-1) or (b-1) is less than 30, the cross-link density may exceed the moderate density, so the polymer composition obtained decreases it's absorption capacity.

The polymer composition having high swelling rate and high fluid stable aggregate content can be preferably obtained by reacting the absorbent resin powder (A) with the cross-linking agent (B) until the reaction is completed i.e., until the time when the equation (a-1) or (b-1) is satisfied.

The reaction of the abosrbent resin powder (A) with the cross-linking agent (B) proceeds after the mixing of the abosrbent resin powder (A) with the cross-linking agent (B). The reaction necessitates application of heat particularly when a polyhydric alcohol, a polyglycidyl compound, a polyamine compound, or a polyoxazoline compound is used as the cross-linking agent (B). The heat treatment is carried out after the abosrbent resin powder (A) and the cross-linking agent (B) have been mixed, at a temperature in the range of 90° to 250°C.

When a polyhydric alcohol is used as the cross-linking agent (B) and the heating temperature is selected in the range of 90° to 250°C, preferably in the range of 170° to 220°C, the cross-linking reaction enough for sufficient manifestation of the effect of this invention can be effected quickly without entailing the possibility of the absorbent resin being colored or deteriorated. It should be noted parenthetically that when the heat treatment is carried out at a high temperature exceeding 250°C, the absorbent resin may succumb to thermal deterioration, depending on the kind of the resin.

In the present invention, the reaction product of the absorbent resin powder (A) with the cross-linking agent (B), when necessary, may be pulverized and granulated. The pulverization and granulation can be carried out by the use of an ordinary pulverizing granulator. The pulverizing granulaters which are usable herein include New Speed Mill (produced by Okada Seiko K.K.), Flush Mill (produced by Fuji Powder K.K.), and Speed Mill (produced by Showa Engineering K.K.), for example.

The polymer composition which is obtained by the method of the present invention has high swelling rate and high fluid stable aggregate content and possesses very little fine powder content. Thus, the present invention solves the various problems encountered by the conventional absorbent resin as described above. Further, there can be obtained a polymer composition which abundantly contains tenacious aggregates of a structure sparingly disintegrable on absorption of liquid. The polymer composition thus containing aggregates in a great abundance particularly exhibits notably improved liquid permeability.

The polymer composition which is obtained by the method of this invention, therefore, is usable as absorbent in sanitary articles such as sanitary napkins and disposable diapers and is also suitable for a wide variety of products such as coagulants for sludge, dew-drop proofing agents for building materials, and water-retaining agents or desiccants for agriculture and horticulture, for example.

The polymer composition obtained by the method in accordance with the present invention contains the above mentioned fluid stable aggregates in an amount of preferably not less than 30 % by weight, more preferably not less than 40 % by weight, especially not less than 50 % by weight, and shows excellent swelling rate in addition to decrease of fine particles.

An indication that crosslink bonds are being formed between the polymer chains of the previously independent absorbent resin powder particle is that the resultant fluid stable aggregates are fluid (i.e., liquid) stable. "Fluid stable" is used herein to mean an aggregate unit that upon contact with or swelling (with and/or without stress) in an aqueous fluid remains substantially intact (i.e., at least two of the previously independent component absorbent resin powder particles remain joined together). While the definition of fluid stability recognizes that at least two of the absorbent resin powder particles remain joined together, preferably all of the absorbent resin powder particles used to make up the specific fluid stable aggregate remain intact. However, it should be recognized that some of the absorbent resin powder particles may dissociate themselves from the fluid stable aggregate if, for example, certain particles have been subsequently water agglomerated to the fluid stable aggregate.

Fluid stability of the fluid stable aggregates of the present invention allows the fluid stable aggregate to maintain its structure in both the dry and wet (swollen) state, to immobilize the component absorbent resin powder particles to minimize migration of the particles, and to maintain a rapid rate of fluid uptake. In an end product such as an absorbent member, fluid stability is beneficial in reducing gel blocking since the precursor particles remain aggregated even when contacted with excess liquids, in allowing one to use previously independent fine particles in an aggregated form, and in increasing the rate of fluid uptake of the resultant polymeric composition without introducing the element of gel blocking. Further, the larger particles of the fluid stable aggregates open the absorbent member's capillary channels providing improved liquid handling characteristics. The fluid stability of aggregates can be determined by the following process. A relatively large particle (i.e., more than 300 microns in size) with aggregate characteristics (i.e., comprising a multiplicity of absorbent resin powder particles) is selected. An aqueous fluid (Synthetic Urine) is added to the aggregate particle, and then the fully swollen equilibrium condition of the aggregate is observed.

A particle is considered unstable if the aggregate particle has a large number of broken away component absorbent resin powder particles. The main aggregate particle (if it still exists) is carefully moved with spatula to determine whether particles have separated from the main aggregate particle. If the main aggregate particle breaks apart upon gentle probing or there are number of particles broken off, the particle is considered unstable. If the aggregate particle remains relatively stable after each test procedure, the aggregate particle is considered stable.

Now, the present invention will be described more specifically below with reference to working examples. It should be noted, however, that this invention is not limited to these examples. In the examples, the term "%" means "% by weight" and the term "part" means "part by weight" unless otherwise specified.

Example 1

A jacketed twin arm type kneader of stainless steel measuring 10 liters in inner volume, 220 mm x 240 mm in the opening and 240 mm in depth, and provided with two Sigma type blades possessing a rotational diameter of 120 mm was stoppered with a lid. Into this kneader, a monomer component containing 5,500 g of an aqueous solution of sodium acrylate possessing a neutralization ratio of 75 mol% and 3.4 g of trimethylol propane triacrylate (0.05 mol% based on sodium acrylate possessing a neutralization ratio of 75 mol%) (the monomer concentration 37% by weight in the aqueous solution) was introduced and nitrogen gas was blown to displace the air entrapped inside the reaction system. Then the Sigma type blades were set rotating at rates of 46 rpm and, at the same time, the jacket was heated by passage of hot water at 35°C. As a polymerization initiator, 2.8 g of sodium persulfate and 0.14 g of L-ascorbic acid were added. Polymerization started four minutes after the addition of the polymerization initiator. The peak temperature inside the reaction system reached 82°C after the elapse of 15 minutes following the addition of the polymerization initiator. The hydrated gel polymer had been divided into minute particles about 5 mm in size. The stirring was further continued. The lid was removed form the kneader 60 minutes after the start of the polymerization and the gel was removed form the kneader.

The minute particles of hydrated gel polymer thus obtained were spread on a 50-mesh metal gauze and dried with hot air at 150°C for 90 minutes. The dried minute particles of hydrated gel polymer were pulverized with a hammer type crusher and sifted with a 20-mesh metal gauze to obtain a 20-mesh pass portion [absorbent resin powder (A-1)].

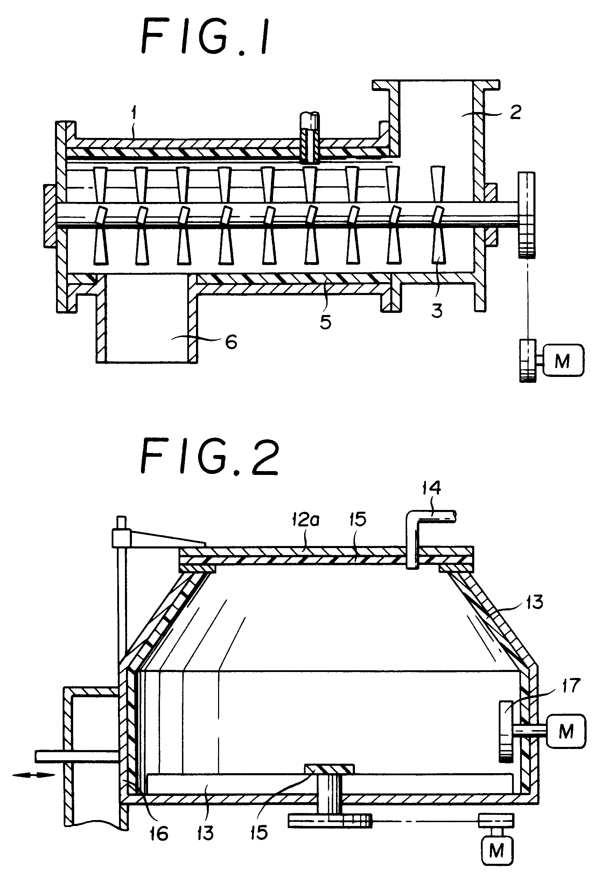

In a Turbulizer 1 (produced by Hosokawa Micron K.K.) fitted with an inner tube 5 made of polytetrafluoroetylene (contact angle 114° and heat distortion point 121°C) having 10 mm in thickness as shown in fig. 1, the absorbent resin powder (A-1) was charged continuously from a powder inlet 2 and a liquid mixture of glycerol and water was continuously charged from a liquid inlet 4 at a rate of 2 parts of glycerol and 4 parts of water per 100 parts of the absorbent resin powder (A-1) and the mixture was mixed. The leading-end peripheral speed of the stirring blades 3 in the Turbulizer 1 was 1280 m/minutes. 700 g of the resultant mixture discharged from an outlet 6 was charged into a bowl dipped in an oil bath (220°C) and was subjected to heat-treatment for 80 minutes under stirring (60 rpm) by a mortar mixer (produced by Nishinihon Testing Machine Co.) to obtain an aggregate polymer composition. Input electric power was measured by a power analyzer PA-1000 (produced by K.K. Musashi Denki Keiki Seisakusho), it was stable at 165 W. On the other hand, the same mortar mixer was operated for 80 minutes without said mixture, the input electric power was stable at 163 W. Efficiency was read from a characteristic table of the motor and a kinetic energy loaded to the mixture during the crosslinking reaction was calculated as follows:

$$F = 4.8 \text{ W} \cdot \text{hr/kg} = 17,280 \text{ joule/kg}$$

The resultant aggregate polymer composition was pushed through a 18-mesh metal gauze (ASTM), to obtain fluid stable aggregate (FSA) (1).

The absorbent resin powder (A-1) and the FSA (1) obtained as described above were tested for (i) absorption capacity, (ii) swelling rate, (iii) FSA content and (iv) particle size distribution as follows. The results were as shown in Table 1.

(i) Absorption capacity: A pouch (40 mm x 150 mm) made of non-woven fabric after the fashion of a tea bag and filled evenly with about 0.2 g of a sample of abosrbent resin powder (A-1) or fluid stable aggregate (1) was immersed in an aqueous 0.9 % NaCl solution for 60 minutes, removed from the solution, left draining for 5 seconds, further removing water on a 24 folded toilet paper having 60 cm for 10 seconds, and weighed.

$$\text{Absorption capacity (g/g)} = \frac{\text{Weight after absorption (g) - Blank (g)}}{\text{Weight of sample polymer (g)}}$$

(ii) Swelling rate: The sample of abosrbent resin powder (A-1) or fluid stable aggregate (1) was riffled according to manufacturers direction and sieved to obtain a through 20 mesh (850 micron) on 30 mesh (600 micron) cut or a through 30 mesh on 50 mesh (300 micron) cut.

0.450 Grams of the sample 20/30 cut or 30/50 cut was weighed and placed in the bottom of a standard 0.5 inch diameter test tube. 12.6 Grams of Jayco synthetic urine (containing 0.2 % of KCl, 0.2 % of $Na_2SO_4$, 0.085 % of $(NH_4)H_2PO_4$, 0.015 % of $(NH_4)_2HPO_4$, 0.025 % of $CaCl_2 \cdot 2H_2O$ and 0.050 % of $MgCl_2 \cdot 6H_2O$) was added to the vertically supported test tube, while at the same time activating a stopwatch.

The watch was stopped at the moment when the rising gel mass reached the bottom of the fluid meniscus in the tube. 28 Grams per gram synthetic urine per gram of sample polymer was divided by the time elaspsed in seconds to obtain the swelling rate.

(iii) FSA content: The sample of absorbent resin powder (A-1) or fluid stable aggregate (1) was riffled according to manufacturers direction and sieved to obtain a through 20 mesh (850 micron) on 30 mesh (600 micron) out or a through 30 mesh on 50 mesh (300 micron) out.

The FSA content was determined by taking 50 particles from the 20/30 cut, freeing the particles of single particles, dropping about 0.1 g of synthetic urine on each of the remaining particles, that is aggregates, and allowing the particles to absorb the synthetic urine for 10 minutes, then removing the excess synthetic urine with gauzy paper, finding the number (m) of gel particles not divided into two or more gel particle fragments, and finding the number (n) by repeating the procedure described above on the particles taken from the 30/50 cut.

$$\text{FSA content (\%)} = \{(m + n)/(50 + 50)\} \times 100$$

(iv) Particle size distribution: Standard sieves 20 mesh, 50 mesh, and 100 mesh in size (70 mm in diameter) and a receiving plate as a classifying plate were superposed. In the uppermost sieve, 30 g of a sample of absorbent resin powder (A-1) or fluid stable aggregate (1) was placed. The composite sieve was shaken by a classifier for 10 minutes. The portion of the sample collected in the classifying plate was weighed and reported in % by weight.

(v) Contact angle of the substrate: It was measured by a liquid dropping method using a contact angle meter CA-DT-A type (manufactured by Kyowa Interface Science Co., Ltd.).

(vi) Heat distortion point of the substrate: It was measured by a method of ASTM D-648 (4.6 kg $\cdot$ cm$^{-2}$).

(vii) Calculated Value of Formula: Water content (105°C, 3hrs) of the absorbent powder (A-1) was 2% (wet basis), the value P/0.98 = P′ was inserted into the equation (a-1) to calculate the value of the equation. Further, water content of fluid stable aggregate (1) was 0%.

Example 2

A similar method to Example 1 was repeated to obtain FSA (2) except that 500 g of the resultant mixture discharged from an outlet 6 was charged and rotation number of the mortar mixer was 120 rpm. A similar test to Example 1 was carried out and the results were shown in Table 1.

Example 3

100 Parts by weight of the absorbent resin powder (A-1) obtained in Example 1 and 0.3 part by weight of a water-insoluble fine silica ("Aerosil 200", a trade name for a product of Aerosil Co., Ltd.) were mixed by a V-type mixer to obtain an absorbent resin powder B. A similar method to Example 2 was repeated to obtain FSA (3) except that the absorbent resin powder B was used instead of the absorbent resin powder (A-1). A similar test to Example 1 was carried out and the results were shown in Table 1.

Example 4

A similar method to Example 1 was repeated to obtain FSA (4) except that a liquid mixture added 8 parts by weight of isopropanol into 2 parts by weight of glycerol and 4 parts by weight of water was used and heat-treatment time was 40 minutes. A similar test to Example 1 was carried out and the results were shown in Table 1.

Control 1

A similar method to Example 1 was repeated to obtain an absorbent (1) except that the heat-treatment was carried out by using a hot air oven at 80°C instead of the mortar mixer. A similar test to Example 1 was carried out and the results were shown in Table 1.
Agglomeration was recognized, but there was no fluid stable aggregate in the absorbent (1) and swelling rate was extremely low.

Control 2

A similar method to Example 1 was repeated to obtain an absorbent (2) except that 3000 parts by weight of the absorbent resin powder (A-1), 60 parts by weight of glycerol and 120 parts by weight of water were mixed in batchwise by using a kneader coated with tetrafluoroethylene-perfluoroalkyl vinyl ether copolymer on the inner wall (rotation number of blades was 46 rpm) instead of Turbulizer. The leading-end peripheral speed of the blades was 52 m/min. A similar test to Example 1 was carried out and the results were shown in Table 1.

Control 3

A similar method to Example 1 was repeated to obtain an absorbent (3) except that a kneader was used instead of the mortar mixer. A similar test to Example 1 was carried out and the results were shown in Table 1.

Control 4

A similar method to Example 4 was repeated to obtain an absorbent (4) except that a mortar mixer coated with tetrafluoroethylene-perfluoroalkyl vinyl ether copolymer on the inner wall was used instead of Turbulizer. The leading-end peripheral speed of the stirring blades was 92 m/min. A similar test to Example 1 was carried out and the results were shown in Table 1.

Example 5

A similar method to Example 1 was repeated to obtain a FSA (5) except that Turbulizer having an inner tube 5 made of high density polyethylene was used. A similar test to Example 1 was carried out and the results were shown in Table 1.

Example 6

A FSA (6) is obtained by the following procedure of Example 1, except that a HEAVY DUTY MATRIX (Produced by Nara Kikai Seisakusho K.K.) fitted with an inner wall 15 made of polytetrafluoroethylene (contact angle 114° and heat distortion point 121°C) having 10 mm in thickness as shown in Fig. 2 is used instead of Turbulizer. The HEAVY DUTY MATRIX is provided with a powder inlet 12 having a lid 12a and a liquid inlet (2-fluid nozzle) 14, a stirring blade 13 on the bottom, a crushing blade 17 at a side wall, and an outlet 16. The leading-end peripheral speed of the stirring blade 13 is about 700 m/min.

The FSA (6) has a similar physical properties to Example 1.

Example 7

A pulverized hydrated gel was obtained by the following procedure of Example 1, except that 1.7 g of trimethylol propane triacrylate (0.025 mol% based on sodium acrylate possessing a neutralization ratio of 75 mol%) was used. The gel was dried by a similar method as in Example 1, the dried gel was crushed by a hammer type crusher under severer condition than that of Example 1 to obtain powder passed 20-mesh metal gauze [absorbent resin powder (A-2)].

100 parts of the absorbent resin powder (A-2) and a liquid mixture containing 4 parts of glycerol, 8 parts of water and 2 parts of isopropanol was mixed in a similar Turbulizer as in Example 1. 1,000 g of mixture thus obtained was spreaded in a pan (30 cm x 60 cm) and the pan was inserted into a hot air drier and heat-treated at a temperature of 210°C for 30 minutes to obtain aggregate polymer composition. The resultant aggregate polymer composition was pushed through a 18-mesh metal gauze, to obtain a FSA (7).

The absorbent resin powder (A-2) and the FSA (7) were tested by the same method as in Example 1 and the results were shown in Table 1.

Example 8

An absorbent resin powder C was obtained by the following procedure of Example 1, except that dried gel was crushed by a hammer type crusher under severer condition than that of Example 1 to obtain powder passed through 20 mesh metal gauze [absorbent resin powder (A-3)] and 100 parts by weight of the absorbent resin powder (A-3) and 1 part by weight of Aerosil 200 was mixed by a V-type mixer.

A FSA (8) was obtained by the following procedure of Example 1, except that 101 parts by weight of the absorbent resin powder C, and a liquid mixture containing 0.5 part by weight of ethyleneglycol diglycydil ether, 30 parts by weight of water and 4 parts by weight of methanol were mixed in a similar Turbulizer as in Example 1 under the condition described in Table 1. A similar test was carried out and the results were shown in Table 1.

Table1

| | Absorbent Resin Powder A-1 | Example 1 FSA (1) | Example 2 FSA (2) | Example 3 FSA (3) | Example 4 FSA (4) | Control 1 Absorbent (1) | Control 2 Absorbent (2) | Control 3 Absorbent (3) | Control 4 Absorbent (4) | Example 5 FSA (5) |
|---|---|---|---|---|---|---|---|---|---|---|
| Precursor | | A-1 | A-1 | A-1 | A-1 | A-1 | A-1 | A-1 | A-1 | A-1 |
| Absorption Capacity (g/g) | 46 | 46 | 46 | 46 | 46 | 46 | 46 | 46 | 46 | 46 |
| Precursor (Parts) | | 100 | 100 | 100 | 100 | 100 | 100 | 100 | 100 | 100 |
| Crosslinking Agent | | Glycerol | Glycerol | Glycerol | Glycerol | Glycerol | Glycerol | Glycerol | Glycerol | Glycerol |
| Crosslinking Agent (Parts) | | 2 | 2 | 2 | 2 | 2 | 2 | 2 | 2 | 2 |
| Water (Parts) | | 4 | 4 | 4 | 4 | 4 | 4 | 4 | 4 | 4 |
| Hydrophilic Organic Solvent (Parts) | | 0 | 0 | 0 | 8 | 0 | 0 | 0 | 8 | 0 |
| | | | | $SiO_2$ 0.3 | | | | | | |
| Mixer | | Turb | Turb | Turb | Turb | Turb | Kneader | Turb | Mortar | Turb |
| Inner Substrate | | PTFE | PTFE | PTFE | PTFE | PTFE | PFA | PTFE | PFA | HDPE |
| Conract Angle (dgree) | | 114 | 114 | 114 | 114 | 114 | 115 | 114 | 115 | 88 |
| Heat Distortion (°C) | | 121 | 121 | 121 | 121 | 121 | 75 | 121 | 75 | 82 |
| Peri pherol speed (m/min) | | 1280 | 1280 | 1280 | 1280 | 1280 | 52 | 1280 | 92 | 1280 |
| Heater | | Mortar/60rpm | Mortar/120rpm | Mortar/120rpm | Mortar/60rpm | Oven | Mortar/60rpm | Kneader/60rpm | Mortar/60rpm | Mortar/60rpm |
| Kinetic Energy (joule/kg) | | 17,280 | 28,800 | 28,800 | 8,640 | 0 | 17,280 | 61,200 | 8,640 | 17,280 |
| Temperature (°C) | | 220 | 220 | 220 | 220 | 80 | 220 | 220 | 220 | 220 |
| Time (min) | | 80 | 80 | 80 | 40 | 80 | 80 | 70 | 40 | 80 |
| FSA Properties | | | | | | | | | | |
| Absorption Capaity (g/g) | 46 | 36 | 38 | 39 | 41 | 46 | 38 | 33 | 41 | 36 |
| Calculated Value of Formula | | 77 | 82 | 84 | 88 | | 82 | 71 | 88 | 77 |
| Swelling Rate #20~30 (g/g/sec) | 0.12 | 0.36 | 0.29 | 0.30 | 0.25 | 0.01 | 0.14 | 0.14 | 0.19 | 0.34 |
| #30~50 | 0.24 | 0.26 | 0.20 | 0.21 | 0.19 | 0.02 | 0.16 | 0.16 | 0.16 | 0.22 |
| FSA Content(%) #20~50 | 0 | 72 | 60 | 62 | 41 | 0 | 8 | 5 | 25 | 70 |
| Particle on #20 | 0 | 3 | 4 | 3 | 4 | 3 | 8 | 1 | 5 | 4 |
| Size #50 | 50 | 64 | 65 | 67 | 66 | 65 | 60 | 58 | 61 | 63 |
| Distribution #100 | 29 | 25 | 25 | 24 | 24 | 24 | 20 | 26 | 24 | 24 |
| (%) thru #100 | 21 | 8 | 6 | 6 | 6 | 8 | 12 | 15 | 10 | 9 |

Table 1 (Continued)

| | | Absorbent Resin Power A-2 | Example 7 FSA(7) | Absorbent Resin Power A-3 | Example 8 FSA(8) |
|---|---|---|---|---|---|
| Precursor | | | A-2 | | A-3 |
| Absorption Capacity | (g/g) | 54 | 54 | 46 | 46 |
| Precursor | (Parts) | | 100 | | 100 |
| Cross linking Agent | | Glycerol | Glycerol | | EGDGE |
| Cross linking Agent | (Parts) | | 4 | | 0.5 |
| Water | (Parts) | | 8 | | 30 |
| Hydrophilic Organic Solvent(Parts) | | | 2 | | 4 |
| | | | | | $SiO_2$ 1 |
| Mixer | | | Turb | | Turb |
| Inner Substrate | | | PTFE | | PTFE |
| Contact Angle | (dgree) | | 114 | | 114 |
| Heat Distortion | (°C) | | 121 | | 121 |
| Peripheral speed | (m/min) | | 1280 | | 1280 |
| Heater | | | Oven | | Oven |
| Kinetic Energy | (joule/kg) | | 0 | | 0 |
| Temperature | (°C) | | 210 | | 180 |
| Time | (min.) | | 30 | | 40 |
| FSA Properties | | | | | |
| Absorption Capasity | (g/g) | 54 | 36 | 46 | 37 |
| Calculated Valud of Formula | | | 67 | | 79 |
| Swelling Rate | #20~30 | | 1.1 | | 0.31 |
| (g/g/sec) | #30~50 | 0.21 | 0.64 | 0.24 | 0.20 |
| FSA Content(%) | #20~50 | 0 | 100 | 0 | 58 |
| Particle | on#20 | 0 | 17 | 0 | 8 |
| Size | #50 | 5 | 50 | 9 | 28 |
| Distribution | #100 | 34 | 22 | 39 | 51 |
| (%) | thru#100 | 61 | 11 | 52 | 13 |

## Claims

1. A method for the production of a fluid stable aggregate, which comprises mixing (A) 100 parts by weight of an absorbent resin powder possessing a carboxyl group, (B) 0.01 to 30 parts by weight of a cross-linking agent having at least two functional groups capable of reacting with the carboxyl group of the absorbent resin powder, (C) 0 to 50 parts by weight of water, and (D) 0 to 60 parts by weight of a hydrophilic organic solvent in a high-speed stirring type mixer; the mixer having an inner surface formed substantially of a substrate (I) possessing a contact angle of not less than about 60° with respect to water and a heat distortion point of not lower than about 70°C under stirring conditions in which the leading-end of a stirring blade has a peripheral speed of not less than 600 m/min, and thereafter completing reaction of the absorbent resin powder (A) with the cross-linking agent (B) at a temperature in the range of 90° to 250°C under the condition that the total kinetic energy F added to the mixture during the reaction is satisfied by the following equation:

$$0 \leqq F \leqq 36{,}000 \text{ joule/kg,}$$

and in which the kinetic energy Fa per minute added during the reaction is not more than 600 joule/kg.

2. A method as claimed in Claim 1, characterised in that the peripheral speed of the leading-end of the stirring blade is in the range of 1,000 to 3,000 m/min.

3. A method as claimed in Claim 1 or Claim 2, characterised in that the inner surface formed substantially of the substrate (I) of the high-speed stirring type mixer has a thickness of not less than 5mm.

4. A method as claimed in Claim 1 or Claim 2, characterised in that the surface is shaped from the material of substrate (I) and detachably inserted into the mixer.

5. A method as claimed in Claim 4, characterised in that the shaped material is cylindrical.

6. A method as claimed in any preceding Claim, characterised in that the substrate (I) is selected from polyethylene, polypropylene, polyesters, polyamides, fluorine resin, polyvinyl chloride, epoxy resin, and silicone resin.

7. A method as claimed in any preceding Claim, characterised in that the cross-linking agent (B) is selected from polyhydric alcohol compounds, polyglycidyl ether compounds, polyoxazoline compounds, and polyamine compounds.

8. A method as claimed in any preceding Claim, characterised in that the cross-linking agent (B) is used in an amount in the range of 0.1 to 10 parts by weight, based on 100 parts by weight of the absorbent resin powder (A).

9. A method as claimed in any preceding Claim, characterised in that the water (C) is used in an amount in the range of 0.5 to 40 parts by weight, based on 100 parts by weight of the absorbent resin powder (A).

10. A method as claimed in any preceding Claim, characterised in that the hydrophilic organic solvent (D) is used in an amount in the range of 0.1 to 10 parts by weight, based on 100 parts by weight of the absorbent resin powder (A).

11. A method as claimed in Claim 7, characterised in that the agent (B) is a polyhydric alcohol compound and the reaction temperature is in the range of 150° to 250°C.

12. A method as claimed in any preceding Claim, characterised in that the mixing of the components (A) to (D) is carried out continuously.

13. A method as claimed in any of Claims 1 to 7, characterised in that the mixing of the absorbent resin powder (A) with the cross-linking agent (B) is carried out in the presence of 0.01 to 10 parts by weight of water-insoluble fine powder (E), based on 100 parts by weight of the absorbent resin powder (A).

14. A method as claimed in Claim 13, characterised in that the cross-linking agent (B) is used in an amount in the range of 0.1 to 10 parts by weight, the water (C) is used in an amount in the range of 0.5 to 40 parts by weight, the hydrophilic organic solvent (D) is used in an amount in the range of 0 to 20 parts by weight, and the water-insoluble fine powder (E) is used in an amount in the range of 0.01 to 10 parts by weight, based on 100 parts by weight of the absorbent resin powder (A).

15. A method as claimed in Claim 14, characterised in that the hydrophilic organic solvent (D) is used in an amount in the range of 0.1 to 10 parts by weight, based on 100 parts by weight of the absorbent resin powder (A).

**16.** A method as claimed in any of Claims 13 to 15, characterised in that the insoluble fine powder (E) is used in the range of 0.01 to 5 parts by weight, based on 100 parts by weight of the absorbent resin powder (A).

**17.** A method as claimed in any of Claims 13 to 16, characterised in that the cross-linking agent (B) is a polyhydric alcohol compound.

**Patentansprüche**

**1.** Verfahren zur Herstellung eines flüssigkeitsstabilen Aggregats durch Vermischen von (A) 100 Gew.-Teilen eines absorbierenden Harzpulvers mit einer Carboxylgruppe, (B) 0,01 bis 30 Gew.-Teilen eines Vernetzungsmittels mit mindestens zwei funktionellen Gruppen die dazu fähig sind, mit der Carboxylgruppe des abosrbierenden Harzpulvers zu reagieren, (C) 0 bis 50 Gew.-Teilen Wasser und (D) 0 bis 60 Gew.-Teilen eines hydrophilen organischen Lösungsmittels in einem Hochgeschwindigkeitsrührmischer, wobei der Mischer eine innere Oberfläche hat, die im wesentlichen aus einem Substrat (I) gebildet wird, das einen Kontaktwinkel von nicht weniger als etwa 60° in Bezug auf Wasser und einen Wärmeverformungspunkt von nicht weniger als etwa 70°C unter Rührbedingungen hat, wobei das Hauptende der Rührschaufel eine Umfangsgeschwindigkeit von nicht weniger als 600 m/min hat und wobei anschließend die Reaktion des absorbierenden Harzpulvers (A) mit dem Vernetzungsmittel (B) bei einer Temperatur rm Bereich von 90° bis 250°C vervollständigt wird, unter der Bedingung, daß die gesamte Kinetische Energie F, die der Mischung während der Reaktion zugeführt wird, der folgenden Formel genügt:

$$0 \leq F \leq 36\,000 \text{ Joule/kg},$$

und wobei die kinetische Energie Fa pro Minute, die während der Reaktion zugeführt wird, nicht mehr als 600 Joule/kg beträgt.

**2.** Verfahren gemäß Anspruch 1, dadurch gekennzeichnet, daß die Umfangsgeschwindigkeit des Hauptendes der Rührschaufel im Bereich von 1.000 bis 3.000 m/min liegt.

**3.** Verfahren gemäß Anspruch 1 und/oder Anspruch 2, dadurch gekennzeichnet, daß die innere Oberfläche des Hochgeschwindigkeitsrührmischers, die im wesentlichen aus dem Substrat (I) gebildet ist, eine Dicke von nicht weniger als 5 mm hat.

**4.** Verfahren gemäß Anspruch 1 und/oder Anspruch 2, dadurch gekennzeichnet, daß die Oberfläche aus dem Material des Substrats (I) geformt ist und auswechselbar in den Mischer eingesetzt wird.

**5.** Verfahren gemäß Anspruch 4, dadurch gekennzeichnet, daß das geformte Material zylindrisch ist.

**6.** Vefahren gemäß einem oder mehreren der vorhergehenden Ansprüche, dadurch gekennzeichnet, daß das Substrat (I) ausgewählt wird aus Polyethylen, Polypropylen, Polyestern, Polyamiden, Fluorharz, Polyvinylchlorid, Epoxidharz, sowie Siliconharz.

**7.** Verfahren gemäß einem oder mehreren der vorhergehenden Ansprüche, dadurch gekennzeichnet, daß das Vernetzungsmittel (B) ausgewählt wird aus mehrwertigen Alkoholverbindungen, Polyglycidyletherverbindungen, Polyoxazolinverbindungen, sowie Polyaminverbindungen.

**8.** Verfahren gemäß einem oder mehreren der vorhergehenden Ansprüche, dadurch gekennzeichnet, daß das Vernetzungsmittel (B) in einer Menge im Bereich von 0,1 bis 10 Gew.-Teilen, bezogen auf 100 Gew.-Teile des absorbierenden Harzpulvers (A) verwendet wird.

**9.** Verfahren gemäß einem oder mehreren der vorhergehenden Ansprüche, dadurch gekennzeichnet, daß das Wasser (C) in einer Menge im Bereich von 0,5 bis 40 Gew.-Teilen, bezogen auf 100 Gew.-Teile des absorbierenden Harzpulver (A) verwendet wird.

**10.** Verfahren gemäß einem oder mehreren der vorhergehenden Ansprüche, dadurch gekennzeichnet, daß das hydrophile organische Lösungsmittel (D) in einer Menge im Bereich von 0,1 bis 10 Gew.-Teilen, bezogen auf 100 Gew.-Teile des absorbierenden Harzpulvers (A) verwendet wird.

**11.** Verfahren gemäß Anspruch 7, dadurch gekennzeichnet, daß das Mittel (B) eine mehrwertige Alkoholverbindung ist und die Reaktionstemperatur im Bereich von 150° bis 250°C liegt.

**12.** Verfahren gemäß einem oder mehreren der vorhergehenden Ansprüche, dadurch gekennzeichnet, daß das Vermischen der Komponenten (A) bis (D) kontinuierlich durchgeführt wird.

**13.** Verfahren gemäß einem oder mehreren der Ansprüche 1 bis 7, dadurch gekennzeichnet, daß das Vermischen des absorbierenden Harzpulvers (A) mit dem Vernetzungsmittel (B) in Gegenwart von 0,01 bis 10 Gew.-Teilen eines wasserunlöslichen feinen Pulvers (E), bezogen auf 100 Gew.-Teile des absorbierenen Harzpulvers (A) durchgeführt wird.

**14.** Verfahren gemäß Anspruch 13, dadurch gekennzeichnet, daß das Vernetzungsmittel (B) in einer Menge im Bereich von 0,1 bis 10 Gew.-Teilen verwendet wird, das Wasser (C) in einer Menge im Bereich von 0,5 bis 40 Gew.-Teilen verwendet wird, das hydrophile organische Lösungsmittel (D) in einer Menge im Bereich von 0 bis 20 Gew -Teilen verwendet wird und das wasserurlösliche feine Pulver (E) in einer Menge im Bereich von 0,01 bis 10 Gew.-Teilen verwendet wird, bezogen auf 100 Gew.-Teile des absorbierenden Harzpulvers (A).

**15.** Verfahren gemäß Anspruch 14, dadurch gekennzeichnet, daß das hydrophile organische Lösungsmittel (D) in einer Menge im Bereich von 0,1 bis 10 Gew.-Teilen, bezogen auf 100 Gew.-Teile des aborbierenden Harzpulvers (A) verwendet wird.

**16.** Verfahren gemäß einem oder mehreren der Ansprüche 13 bis 15, dadurch gekennzeichent, daß das unlösliche feine Pulver (E) in einem Bereich von 0,01 bis 5 Gew.-Teilen, bezogen auf 100 Gew.-Teile des absorbierenden harzpulvers (A) verwendet wird.

**17.** Verfahren gemäß einem oder mehreren der Ansprüche 13 bis 16, dadurch gekennzeichnet, daß das Vernetzungsmittel (B) eine mehrwertige Alkoholverbindung ist.

## Revendications

**1.** Procédé pour la production d'un agrégat fluide stable, qui consiste à mélanger (A) 100 parties en poids d'une poudre de résine absorbante comportant un groupe carboxyle, (B) 0,1 à 30 parties en poids d'un agent de réticulation comportant au moins deux groupes fonctionnels aptes à réagir avec le groupe carboxyle de la poudre de résine absorbante, (C) 0 à 50 parties en poids d'eau et (D) 0 à 60 parties en poids d'un solvant organique hydrophile dans un mélangeur du type à agitation à grande vitesse; le mélangeur comportant une surface intérieure formée essentiellement d'un substrat (I) possédant un angle de contact avec l'eau non inférieur à environ 60° et un point de déformation thermique non inférieur à environ 70°C dans des conditions d'agitation dans lesquelles l'extrémité avant d'une lame d'agitation possède une vitesse périphérique non inférieure à 600 m/mn, puis achever la réaction de la poudre de résine absorbante (A) avec l'agent de réticulation (B) à une température dans la gamme de 90° à 250°C, dans les conditions dans lesquelles l'énergie cinétique totale F ajoutée au mélange pendant la réaction est satisfaite par la relation suivante :

$$0 \leq F \leq 36\,000 \text{ joules/kg,}$$

et selon lequel l'énergie cinétique $F_a$ ajoutée par minute pendant la réaction n'est pas supérieure à 600 joules/kg.

**2.** Procédé selon la revendication 1, caractérisé en ce que la vitesse périphérique de l'extrémité avant de la lame d'agitation se situe dans la gamme de 1000 à 3000 m/mn.

**3.** Procédé selon la revendication 1 ou la revendication 2, caractérisé en ce que la surface intérieure formée essentiellement dans le substrat (1) du mélangeur du type à agitation à grande vitesse n'est pas inférieure à 5 mm.

**4.** Procédé selon la revendication 1 ou la revendication 2, caractérisé en ce que la surface est conformée à partir du matériau du substrat (I) et est insérée de façon amovible dans le mélangeur.

**5.** Procédé selon la revendication 4, caractérisé en ce que le matériau conformé est cylindrique.

**6.** Procédé suivant l'une quelconque des revendications précédentes, caractérisé en ce que le substrat (I) est choisi parmi le polyéthylène, le polypropylène, des polyesters, des polyamides, une résine fluorée, du chlorure de polyvinyle, une résine époxy et une résine silicone.

7. Procédé suivant l'une quelconque des revendications précédentes, caractérisé en ce que l'agent de réticulation (B) est choisi parmi les composés de polyalcools, des composés de polyglycidyléther, des composés de polyoxazoline et des composés de polyamine.

8. Procédé suivant l'une quelconque des revendications précédentes, caractérisé en ce que l'agent de réticulation (B) est utilisé en une quantité dans la gamme de 0,1 à 10 parties en poids, sur la base de 100 parties en poids de la poudre de résine absorbante (A).

9. Procédé suivant l'une quelconque des revendications précédentes, caractérisé en ce que l'eau (C) est utilisée en une quantité située dans la gamme de 0,5 à 40 parties en poids, sur la base de 100 parties en poids de la poudre de résine absorbante (A).

10. Procédé suivant l'une quelconque des revendications précédentes, caractérisé en ce que le solvant organique hydrophile (D) est utilisé en une quantité située dans la gamme de 0,1 à 10 parties en poids, sur la base de 100 parties en poids de la poudre de résine absorbante (A).

11. Procédé selon la revendication 7, caractérisé en ce que l'agent (B) est un composé de polyalcool et que la température de réaction se situe dans la gamme de 150° à 250°C.

12. Procédé suivant l'une quelconque des revendications précédentes, caractérisé en ce que le mélange des composants (A) à (D) est exécuté continûment.

13. Procédé suivant l'une quelconque des revendications 1 à 7, caractérisé en ce que le mélange de la poudre de résine absorbante (A) avec l'agent de réticulation (B) est exécuté en présence de 0,01 à 10 parties en poids d'une fine poudre (E) insoluble à l'eau, sur la base de 100 parties en poids de la poudre de résine absorbante (A).

14. Procédé selon la revendication 13, caractérisé en ce que l'agent de réticulation (B) est utilisé en une quantité dans la gamme de 0,1 à 10 parties en poids, l'eau (C) est utilisée en une quantité dans la gamme de 0,5 à 40 parties en poids, le solvant organique hydrophile (D) est utilisé dans une gamme de 0 à 20 parties en poids, et la fine poudre insoluble à l'eau (E) est utilisée en une quantité dans la gamme de 0,1 à 10 parties en poids, sur la base de 100 parties en poids de la poudre de résine absorbante (A).

15. Procédé selon la revendication 14, caractérisé en ce que le solvant organique hydrophile (D) est utilisé en une quantité dans la gamme de 0,1 à 10 parties en poids, sur la base de 100 parties en poids de la poudre de résine absorbante (A).

16. Procédé suivant l'une quelconque des revendications 13 à 15, caractérisé en ce que la fine poudre insoluble (E) est utilisée dans la gamme de 0,01 à 5 parties en poids, sur la base de 100 parties en poids de la poudre de résine absorbante (A).

17. Procédé suivant l'une quelconque des revendications 13 à 16, caractérisé en ce que l'agent de réticulation (B) est un composé de polyalcool.

# FIG.1

# FIG.2